## Europäisches Patentamt

## European Patent Office

## Office européen des brevets

(11) Veröffentlichungsnummer: **0 460 674 A2**

# EUROPÄISCHE PATENTANMELDUNG

(21) Anmeldenummer: 91109303.7

(22) Anmeldetag: 06.06.91

(51) Int. Cl.5: **C12N 15/13, C12P 21/08, C07K 15/28**

The microorganism(s) has (have) been deposited with American Type Culture Collection under numbersCRL 1581, CCL 77, CRL 1484, CRL 1580.

(30) Priorität: 08.06.90 DE 4018442

(43) Veröffentlichungstag der Anmeldung:
11.12.91 Patentblatt 91/50

(84) Benannte Vertragsstaaten:
AT BE CH DE DK ES FR GB GR IT LI LU NL SE

(71) Anmelder: **BOEHRINGER MANNHEIM GMBH**
**Sandhofer Strasse 112-132**
**W-6800 Mannheim-Waldhof(DE)**

(72) Erfinder: **Weidle, Ulrich H., Dr.**
**Landwehrstrasse 56**
**W-8000 München 2(DE)**
Erfinder: **Kaluza, Brigitte, Dr.**
**Hochfeldanger 3**
**W-8173 Bad Heilbrunn(DE)**
Erfinder: **Knapp, Walter, Dr.**
**Riedstrasse 16**
**A-1140 Wien(AT)**

(74) Vertreter: **Huber, Bernhard, Dipl.-Chem. et al**
**Möhlstrasse 22 Postfach 860 820**
**W-8000 München 86(DE)**

(54) **Rekombinante DNA und Verfahren zur Herstellung chimärer Antikörper.**

(57) Eine rekombinante DNA, welche für die leichte oder die schwere Kette eines Antikörpers kodiert, dessen konstante Regionen humanen Ursprungs sind und dessen variable Regionen nicht humanen Ursprungs sind, und die in Richtung der Transkription

(a) eine für die variable nicht-humane Region kodierende cDNA-Sequenz, einschließlich der Region J und gegebenenfalls D,

(b) eine Intron-Sequenz, welche an ihrem 5'-Ende eine Splice-Donor-Stelle aufweist und zusammengesetzt ist aus einer nicht-humanen Intron-Teilsequenz am 5'-Ende und einer humanen Intron-Teilsequenz am 3'-Ende, und

(c) eine für die humane konstante Region kodierende genomische DNA-Sequenz

aufweist.

EP 0 460 674 A2

Die Erfindung betrifft rekombinante DNA, welche für die leichte oder schwere Kette eines Antikörpers kodiert, dessen konstante Regionen humanen Ursprungs sind und dessen variable Regionen nicht humanen Ursprungs sind, Expressionsvektoren, enthaltend eine solche rekombinante DNA, sowie Verfahren zur Herstellung der rekombinanten DNA bzw. der Expressionsvektoren und schließlich Verfahren zur Herstellung des chimären Antikörpers.

Die Anwendung von Antikörpern in der Therapie gewinnt immer größere Bedeutung. So sind beispielsweise Antikörper gegen den Interleukin 2 (IL-2)-Rezeptor als Immunsuppressiva von großem therapeutischen Interesse. Die in vivo-Wirksamkeit solcher Antikörper konnte in einer Reihe von experimentellen Tiermodellen belegt werden. Beispiele hierfür sind für Herz- und Haut-Transplantation bei der Maus von Kirkman et al., Transplantation Proceedings 19 (1987) 618-690 und T. Diamantstein et al., Transplantation Reviews 1 (1987), 177-196, für Herz-Transplantation bei der Ratte von J.W. Kupiec-Weglinski et al., Proc. Natl. Acad. Sci. USA 83 (1986) 2624, bei der Transplantation von Langerhans'schen Inseln bei der Ratte von Hahn et al., Diabetologia 30 (1987), 40 und für die Nieren-Transplantation bei Primaten von T. Diamantstein et al., Transplantation Reviews 1 (1987) 177-196 beschrieben. Auch beim Menschen wurde der erfolgreiche präventive Einsatz von Ratten-Antikörpern gegen den IL-2-Rezeptor bei Nieren-Transplantationen beschrieben (J.P. Soulillou et al., J. of Autoimmunity 1 (1988) 655-661; D. Cantarovitch et al., Am. J. of Kidney Disease 11 (1988) 101-106.

Es besteht jedoch ein Nachteil dahingehend, daß Maus- und Ratten-Antikörper nach Applikation im Menschen stark immunogen sind, was zu einer Neutralisierung der therapeutischen Aktivität führen kann. Es besteht daher ein großes Interesse an der Verfügbarkeit von hybriden Maus/Mensch-Antikörpern oder humanisierten Antikörpern, von denen man sich eine wesentlich reduzierte oder gar eliminierte Immunogenität im Menschen verspricht (S.L. Morrison et al., Proc. Natl. Acad. Sci. USA 81 (1984) 6851-6855; P.T. Jones et al., Nature 321 (1986) 522-525.

Derartige hybride Antikörper werden bisher meist durch gentechnologische Fusion entsprechender cDNA's und Expression in geeigneten Wirtszellen hergestellt. Dabei ergibt sich jedoch das Problem, daß die die hybriden Antikörper kodierenden cDNA's schlecht exprimiert werden. Bei einer anderen Möglichkeit der Herstellung werden zur Konstruktion solcher Antikörper üblicherweise zunächst die Gene für die leichte und schwere Kette aus einer Phagen-Bibliothek isoliert und charakterisiert. Anschließend erfolgt die gentechnologische Fusion der genomischen DNA der VJ-Region bzw. der VDJ-Region mit der entsprechenden genomischen DNA der konstanten Regionen der leichten und schweren humanen Ketten (vgl. hierzu S.L. Morrison et al., Proc. Natl. Acad. Sci. USA 81 (1984) 6851-6855; S.H. Boulianne et al., Nature 312 (1984) 641-646; L.K. Sun et al., Proc. Natl. Acad. Sci. USA 84 (1987) 214-218; Y. Nishimura et al., Cancer Res. 47 (1987) 999-1005; B.A. Brown et al., Cancer Res. 47 (1987) 3577-3583).

Da in der Regel $10^6$ Plaques zur Auffindung eines Gens mit Kopienzahl 1 durchgemustert werden müssen, ist diese zweite Art des Vorgehens extrem arbeitsaufwendig. Desweiteren kostet die Charakterisierung der genomischen Isolate (Restriktions-Kartierung, Unterscheidung der Genstruktur in somatischen und Keim-Bahn-Zellen, Unterscheidung von falsch und richtig rearrangierten Genen) viel Zeit.

Aufgabe der vorliegenden Erfindung war es daher, die Herstellung chimärer Antikörper zu erleichtern.

Gelöst wird diese Aufgabe erfindungsgemäß durch eine rekombinante DNA, welche für die leichte oder schwere Kette eines Antikörpers kodiert, dessen konstante Regionen humanen Ursprungs sind und dessen variable Regionen nicht humanen Ursprungs sind, dadurch gekennzeichnet, daß sie in Richtung der Transkription

(a) eine für die variable nicht-humane Region kodierende cDNA-Sequenz, einschließlich der Region J und gegebenenfalls D,

(b) eine Intron-Sequenz, welche an ihrem 5'-Ende eine Splice-Donor-Stelle aufweist und zusammengesetzt ist aus einer nicht-humanen Intron-Teilsequenz am 5'-Ende und einer humanen Intron-Teilsequenz am 3'-Ende, und

(c) eine für die humane konstante Region kodierende genomische DNA-Sequenz
aufweist.

Mit Hilfe der rekombinanten DNA lassen sich in überraschend hoher Ausbeute die leichten bzw. schweren Ketten für chimäre Antikörper der gewünschten Spezifität erzeugen. Es ist hierbei aber außerdem von ganz besonderem Vorteil, daß die leicht zu gewinnende cDNA-Sequenz für die variable Region gewünschter Spezifität (cDNA für Antikörper-Gene ist leicht herzustellen, da die entsprechende mRNA in Hybridomzellen mit großem Anteil an der Gesamt-mRNA vorhanden ist) hier mit einem bereits subklonierten genomischen DNA-Segment, das für die konstanten humanen Regionen kodiert und damit eine breite Anwendbarkeit aufweist, über die Intron-Sequenz verbunden werden kann. Es wird erfindungsgemäß also eine Möglichkeit eröffnet, mit geringem Aufwand rekombinante DNA-Moleküle herzustellen, welche jeweils die DNA-Sequenz für die entsprechende Spezifität aufweisen.

Die cDNA-Sequenz a) der erfindungsgemäßen rekombinanten DNA umfaßt für eine leichte Kette die V- und die J-Region, für eine schwere Kette zusätzlich die D-Region zwischen V und J. Die erfindungsgemäße rekombinante DNA enthält mit ihren Elementen a), b) und c) an allen Stellen, an denen in Antikörpergenen natürlicherweise Introns vorhanden sind, entsprechende DNA-Sequenzen, außer dort, wo Introns in allen leichten und schweren Ketten in der Signalsequenz vorkommen. Die Expression der erfindungsgemäßen rekombinanten DNA in geeigneten Wirtszellen führt zu einer sehr viel höheren Expression der gewünschten Antikörper, verglichen zu bisher bekannten Verfahren zur Herstellung chimärer Antikörper unter Anwendung von cDNA-Expression.

In einer bevorzugten Ausführungsform der Erfindung ist die Sequenz a) ebenso wie die nicht humane Intron-Teilsequenz von b) murinen Ursprungs, und vorzugsweise ist die nicht humane Intron-Teilsequenz von b) 15 bis 20 Nukleotide lang.

In einer besonders bevorzugten Ausführungsform ist die nichthumane Intron-Teilsequenz von b) ein Teil der auf die Sequenz a) natürlicherweise, d.h. in der genomischen DNA, folgenden Intron-Sequenz. Es können jedoch im Rahmen der Erfindung auch andere nicht humane Intron-Sequenzen, vorzugsweise jedoch von der gleichen Spezies und möglichst auch aus einem Antikörpergen abgeleitet, verwendet werden.

In einer weiteren bevorzugten Ausführungsform der Erfindung weist die Splice-Donor-Stelle in b) die Sequenz GT auf.

Erfindungsgemäß ist vorzugsweise eine branch-site im humanen Teil der Intronsequenz gelegen, welche z.B. einer der bekannten Sequenzen, wie sie von Sharp, P.A., Science 235 (1987) 766-771 beschrieben sind, entsprechen kann. Die branch site kann aber auch im murinen Anteil des Introns gelegen sein.

Um die mögliche Expressionsrate der rekombinanten DNA zu steuern, wird vorzugsweise am 5'-Ende der rekombinanten DNA noch eine Promotor-Sequenz angefügt, unter deren Kontrolle die für die Antikörperketten kodierenden Sequenzen exprimierbar sind. Hierbei können z.B. die natürlicherweise, also wie im Genom, die Kontrolle über die entsprechenden Sequenzen der rekombinanten DNA ausübenden Promotoren verwendet werden. Vorzugsweise werden jedoch Promotoren verwendet, die eine verstärkte, gegebenenfalls regulierbare Expression ermöglichen. Derartige Promotoren sind dem Fachmann bekannt. In einer besonders bevorzugten Ausführungsform weist die rekombinante DNA den Promotor des Cytomegalie-Virus auf.

Wiederum eine weitere bevorzugte Ausführungsform der Erfindung liegt in einer rekombinanten DNA, deren Sequenz a) für die variable Region der leichten oder schweren Kette eines mit dem Interleukin-2-Rezeptor spezifisch bindefähigen Antikörpers kodiert. Weiter ist es bevorzugt, daß eine für eine schwere Kette kodierende humane Sequenz c) der erfindungsgemäßen rekombinanten DNA für die konstante Region der schweren Kette eines humanen Antikörpers vom Typ IgG kodiert.

Solche rekombinanten DNA's sind in den Plasmiden pK chim. und pγ chim. enthalten, wobei das Plasmid pK chim. eine für die leichte, nämlich die kappa-Kette kodierende DNA-Sequenz und das Plasmid pγ chim. eine für eine schwere Kette des Typs γ kodierende rekombinante DNA aufweist, welche gemeinsam für einen Antikörper kodieren, dessen Spezifität sich gegen die alpha-Kette des Interleukin-2-Rezeptors richtet und der vom Typ IgG ist.

Ein weiterer Gegenstand der Erfindung ist ein Expressionsvektor, der eine der erfindungsgemäßen rekombinanten DNA's sowie alle zur Expression dieser rekombinanten DNA nötigen Elemente enthält. Diese hierfür notwendigen oder vorzugsweise verwendeten Elemente sind Promotoren, Regulations-Sequenzen und dergleichen. Expressionsvektoren sind dem Fachmann wohl bekannt und beispielsweise von E.L. Winnacker in "Gene und Klone", 1985, Verlag Chemie, Weinheim, näher beschrieben. Mit dem erfindungsgemäßen Expressionsvektor wird es ermöglicht, eine erfindungsgemäße rekombinante DNA in geeignete Wirtszellen einzubringen und dort zur Expression zu bringen. Besonders bevorzugte Expressionsvektoren, welche erfindungsgemäße rekombinante DNA enthalten und ebenfalls Gegenstand der Erfindung, sind die bereits genannten Plasmide pK chim. und pγ chim.

Ein weiterer Gegenstand der Erfindung ist ein Verfahren zur Herstellung einer erfindungsgemäßen rekombinanten DNA, bei dem man aus einer einen Antikörper gewünschter Spezifität sezernierenden Hybridomzellinie polyA$^+$ RNA isoliert, eine cDNA-Bibliothek dazu in geeigneten Vektoren herstellt und über Hybridisierung mit zu für den konstanten Teil des Antikörpers kodierender DNA komplementären Oligonukleotiden auf Klone untersucht, welche die cDNA für Antikörperketten enthalten, hieraus die V-, J- und gegebenenfalls D-Sequenzen isoliert, durch gerichtete Mutagenese anschließend an die jeweiligen J-Domänen in Transkriptionsrichtung eine Splice-Donor-Stelle, einen Teil einer Antikörper-Intronsequenz nicht humanen Ursprungs und eine Restriktionsschnittstelle einführt und durch Verdauung mit dem entsprechenden Restriktionsenzym von den für die konstante Region kodierenden Sequenzen abtrennt, und die

EP 0 460 674 A2

erhaltene DNA mit einer genomischen DNA-Sequenz ligiert, die für die konstante Region der leichten oder schweren Kette eines humanen Antikörpers kodiert und an ihrem 5'-Ende einen Teil einer entsprechenden humanen Intronsequenz aufweist.

Die Isolierung der polyA$^+$ RNA, die cDNA-Synthese hierzu, sowie das Anlegen einer c-DNA-Bibliothek werden nach an sich bekannten Methoden durchgeführt, wie sie z.B. in Maniatis et al., Molecular Cloning: A Laboratory Manual, 1982, Cold Spring Harbor, beschrieben sind. Auch die Auswahl der für Antikörperketten kodierenden cDNA-Klone wird in bekannter Weise durchgeführt, nämlich indem man auf Klone untersucht, die mit einem Oligonukleotid hybridisieren, das komplementär ist zu einer DNA, welche für die konstante Region eines Antikörpers kodiert, der von der gleichen Hybridomzell-Spezies stammt.

Auch die gerichtete Mutagenese zur Einführung der Splice-Donor-Stelle und der Restriktions-Schnittstelle ist an sich bekannt und wird gemäß der Methode, welche von Morinaga et al., Bio/Technology 2 (1984) 636-639 beschrieben wurde, durchgeführt.

In einer bevorzugten Ausführungsform der Erfindung verwendet man eine Maus- oder Ratten-Hybridomzelle. Die Erzeugung von Hybridomzellen, welche Antikörper der gewünschten Spezifität sezernieren, wird nach dem von Köhler und Milstein (Nature 256 (1975), 495) erstmals beschriebenen und nachfolgend weiterentwickelten Verfahren durchgeführt. Diese Arbeiten sind dem auf diesem Gebiet tätigen Fachmann wohlbekannt. Besonders bevorzugt verwendet man im erfindungsgemäßen Verfahren eine Maus-Hybridomzelle, die gegen die alpha-Kette des humanen Interleukin-2-Rezeptors gerichtete Antikörper sezerniert.

Bei der Einführung der nicht humanen Intron-Teilsequenz verwendet man vorzugsweise eine 15 bis 20 Basenpaare lange Sequenz. Besonders bevorzugt verwendet man hier die im authentischen Gen sich anschließende Intronsequenz teilweise. Weiterhin ist es bevorzugt, als Splice-Donor-Stelle die Nukleotidfolge GT einzuführen.

Schließlich ist es erfindungsgemäß weiter bevorzugt, eine humane genomische DNA zu verwenden, deren Intronteilsequenz eine branch-site enthält.

Um eine rekombinante DNA zu erhalten, welche gut exprimiert werden kann, stellt man vorzugsweise der letztlich erhaltenen DNA-Sequenz eine Promotorsequenz voran, oder führt die im erfindungsgemäßen Verfahren nötigen Manipulationen gleich in einem Vektor durch, der in geeigneter Position einen Promotor enthält. Hierbei ist es besonders bevorzugt, den Promotor des Cytomegalie-Virus-Genoms zu verwenden.

In wiederum einer weiteren bevorzugten Ausführungsform der Erfindung verwendet man zur Herstellung einer für eine schwere Kette kodierenden rekombinanten DNA eine humane genomische DNA-Sequenz, die für die konstante Region der schweren Kette eines Antikörpers vom Typ IgG kodiert.

Ein weiterer Gegenstand der Erfindung ist ein Verfahren zur Herstellung eines erfindungsgemäßen Expressionsvektors, bei dem man in einen zur Expression eines Fremdgens geeigneten Vektor eine erfindungsgemäße rekombinante DNA insertiert. Wie bereits oben ausgeführt, sind solche Vektoren dem Fachmann bekannt und enthalten alle notwendigen Elemente wie z.B. Polylinker zum Insertieren der rekombinanten DNA, Antibiotikaresistenz-Gene, um Kolonien auffinden zu können, welche die rekombinante DNA enthalten, einen Replikationsursprung, Regulationselemente, einen Promotor, sofern die rekombinante DNA nicht bereits einen solchen aufweist, sowie gegebenenfalls weitere Elemente, wie z.B. Suppressor-Gene.

Wiederum ein weiterer Gegenstand der Erfindung ist ein Verfahren zur Herstellung eines chimären Antikörpers, dessen variable Regionen nicht humanen Ursprungs und dessen konstante Regionen humanen Ursprungs sind, wobei man hier je einen Expressionsvektor, der eine erfindungsgemäße rekombinante DNA enthält, welche für die leichte Kette und einen Expressionsvektor, der eine erfindungsgemäße rekombinante DNA enthält, welche für die schwere Kette des Antikörpers kodiert, in geeignete Wirtszellen einführt, stabile Transformanten isoliert und die Antikörper nach an sich bekannten Methoden aus dem Kulturüberstand der Zellen gewinnt. Als Wirtszelle wird hierbei vorzugsweise eine Non-Producer-Hybridomzelle verwendet, besonders bevorzugt die Zellinie Sp2/0. Zur Einbringung der Vektoren ist es im erfindungsgemäßen Verfahren bevorzugt, eine Elektroporation (Nucl. Acids Res. 15 (1987), 1311-1326, Bio Techniques 6 (1988), 742-751) durchzuführen, gegebenenfalls mit linearisierten DNA-Molekülen. Es können jedoch auch andere dem Fachmann bekannte Verfahren zur Transfektion der Wirtszellen angewandt werden.

Das erfindungsgemäße Verfahren ermöglicht es, in einfacher Weise Antikörper der verschiedensten Spezifitäten zu erzeugen, welche bei ihrer therapeutischen Anwendung am Menschen nicht oder nur sehr schwach immunogen sind, da die konstanten Regionen humanen Ursprungs sind. Durch die heutzutage relativ einfache Möglichkeit, z.B. Maus-Hybridomzellen herzustellen, welche Antikörper einer gewünschten Spezifität sezernieren, und daraus cDNA zu isolieren, kann diese cDNA nach dem erfindungsgemäßen Verfahren leicht in bereits vorbereitete Vektoren eingebracht werden, die jeweils die genomischen humanen Anteile für die konstanten Regionen der leichten bzw. schweren Kette enthalten. So ist es möglich, durch einfache Integration des gewünschten cDNA-Fragments in die entsprechend vorbereiteten Vektoren chimäre

4

EP 0 460 674 A2

Antikörper der jeweils gewünschten Spezifität nach Expression der rekombinanten DNA beider Vektoren in einer Wirtszelle zu erhalten. Die Herstellung erfolgt nicht nur sehr viel einfacher als mit bisher bekannten Methoden, sondern auch in stark erhöhter Ausbeute.

Ein weiterer Gegenstand der Erfindung sind daher die chimären Antikörper MAK 179, MAK 215 und MAK 447, deren Sequenzen für die leichten und schweren Ketten in den Sequenzprotokollen SEQ ID NO.:1 bis 6 gezeigt sind (angegeben sind die Sequenzen der variablen Regionen, die Sequenzen der konstanten Regionen sind bekannt und z.B. beschrieben in: Sequences of proteins of immunological interest; E. Kabat, T. Wu, M. Reid-Miller, H. Perry und K. Gottesman, US Department of Health and Human Services, 1987, p. 282-325) und deren Herstellung in den Beispielen beschrieben ist. Alle drei Antikörper sind spezifisch für die alpha-Kette des humanen IL 2-Rezeptors.

Die Erfindung wird durch die folgenden Beispiele in Verbindung mit den Sequenzprotokollen und Figuren weiter erläutert.

| | |
|---|---|
| SEQ ID NO.:1 | zeigt die DNA- und Aminosäuresequenz der variablen Region der leichten Kette von MAK 179, |
| SEQ ID NO.:2 | die DNA- und Aminosäuresequenz der variablen Region der leichten Kette von MAK 447 |
| SEQ ID NO.:3 | die DNA- und Aminosäuresequenz der variablen Region der leichten Kette von MAK 215, |
| SEQ ID NO.:4 bis 6 | die hierzu gehörigen Sequenzen der jeweiligen variablen Regionen der schweren Ketten; |
| Fig. 1 | stellt schematisch die Isolierung eines murinen, für die variable Region der leichten Immunglobulinkette kodierenden cDNA-Fragments und die Durchführung der gerichteten Mutagenese dar; |
| Fig. 2a | stellt die Insertion der variablen murinen Region in einen Expressionsvektor dar; |
| Fig. 2b | zeigt schematisch das Zusammenfügen der murinen und humanen Antikörper-kodierenden Sequenzen für die leichte Kette; |
| Fig. 3 und 4a sowie 4b | zeigen dasselbe Vorgehen schematisch für DNA der schweren Immunoglobulin-Ketten. |

Beispiel 1

Klonierung und Sequenz-Analyse der leichten und schweren Ketten von MAK179, 447 und 215

Aus den Hybridom-Linien, die die monoklonalen Antikörper 179, 447 bzw. 215 sezernieren, wurde RNA präpariert (Cleary, et al.: Cell **44** (1986) 97- 106) und daraus über eine Oligo-dT-Cellulose Säule (Collaborative Research, Bedford, MA) poly A$^+$RNA isoliert (Maniatis, T., et al., Molecular Cloning: A Laboratory Manual (Cold Spring Harbor Lab., Cold Spring Harbor, NY, 1982). Mit dem cDNA Klonierungs-Kit von Pharmacia wurde nach den Vorschriften des Herstellers (analog Gubler, U. und Hoffman, G.J.: Gene (1983) 25, 263) eine cDNA Bibliothek für jede Hybridom-Zellinie angelegt. Sie umfaßte für jede Hybridom-Linie etwa 10.000 Rekombinanten. Für das Screenen nach Klonen der kappa-Kette wurde folgender Primer verwendet:

5'CCCGACTACGACGT3'

Für das Screenen nach Klonen der γ-Kette (γ1 und γ2b) wurde folgender Primer verwendet:

5'CAGATAGGTGACCGG3'

Mit diesem Primer werden sämtliche schweren Ketten von Maus Immunglobulin-Genen erfaßt (Sablitzky, F. und Rajewski, K.: EMBO J. 3 (1984) 3005-3012).

Die cDNA Bibliothek wurde im Vektor pT7T318U (Pharmacia) angelegt. Dadurch war es möglich, die Insertionen als EcoRI Fragment wiederzugewinnen. Die Analyse mit Restriktions-Endonukleasen ergab, daß für die leichten und schweren Ketten von MAK179, 447 und 215 Klone der vollen Länge erhalten wurden. Die relevanten Regionen wurden in M13 Vektoren subkloniert und sequenziert (Sanger, F., et al.: Proc. Natl. Acad. Sci. USA 74 (1977) 5463-5467).

Die Sequenzen der variablen Regionen sind in den Sequenzprotokollen SEQ ID NO:1 bis NO:6 dargestellt. Dabei zeigt sich, daß MAK179 und 447 dieselben V-Segmente für die leichten und die schweren Ketten benützen.

Beide kappa-Ketten benützen die J1 Region und besitzen an den VJ Übergängen identische Nukleotide, was auf einen gemeinsamen Ursprung der VJ Region der beiden kappa-Gene hindeutet. Beide γ1 Ketten besitzen dieselbe D-Region, dieselbe J-Region ($J_{H3}$) und identische VDJ Rekombination, was auf

5

einen gemeinsamen Ursprung der VDJ Regionen der beiden γ1 Gene schließen läßt.

Der Vergleich der Aminosäuresequenzen der kappa-Ketten ergibt 3 Aminosäure-Austausche in den V-Regionen der kappa cDNA von MAK179 und MAK447. Es handelt sich um folgende Austausche: (Thr zu Ser, Aminosäure 20), (Lys zu Arg, Aminosäure 45), (Asn zu Lys, Aminosäure 53).

98 % der Aminosäuren der VJ Region von MAK179 und 447 sind identisch.

Es existieren 6 Aminosäure-Austausche in den V-Regionen der γ1 cDNA von MAK179 und MAK447. Es handelt sich um folgende Austausche: (Val zu Ala, Aminosäure 23; Gly zu Ser, Aminosäure 56; Ile zu Val, Aminosäure 58; Thr zu Arg, Aminosäure 63; Lys zu Arg, Aminosäure 65; Gln zu Glu, Aminosäure 82). 94 % der Aminosäuren der VJ Regionen von MAK179 und 447 sind identisch.

Die Daten legen nahe, daß die Unterschiede in den Aminosäure-Sequenzen von MAK179 und MAK447 auf somatische Mutation zurückzuführen sind.

In den Sequenzprotokollen für kappa und γ1 cDNA von MAK179 und 447 sind das Initiationscodon (Met) = Start der Signal-Sequenz angegeben. Ebenfalls angegeben sind die J bzw. D,J Regionen sowie der Beginn der jeweiligen konstanten Regionen.

Die Sequenzen der variablen Regionen für die kappa und die γ2b-Kette des (bezüglich der IL 2-Bindung) nicht-inhibitorischen Antikörpers MAK15 sind dargestellt in SEQ ID No:3 und No:6. Für die kappa-Kette wird die J4 Region benutzt. Es wird ein V-Segment benutzt, das sich von dem der leichten Ketten von MAK179 und 447 stark unterscheidet.

Verglichen mit dem V-Segment von 179 sind 51 % der Aminosäuren identisch.

Die Sequenz der γ2b-Kette ist in SEQ ID NO:6 dargestellt. Es wird ein V-Segment benutzt, welches sich von dem der schweren Kette der Klone MAK179 und MAK447 klar unterscheidet. Die D-Region (umfassend sieben Aminosäuren) sowie die J-Region (J$_{H3}$) und der Beginn der konstanten Region sind angegeben. Verglichen mit dem V-Segment der schweren Kette des Klons MAK179 sind 57 % der Aminosäuren identisch.

Beispiel 2:

<u>Chimärisierung der leichten Kette von MAK179 und Konstruktion eines Expressions-Vektors für die chimärisierte leichte Kette</u>

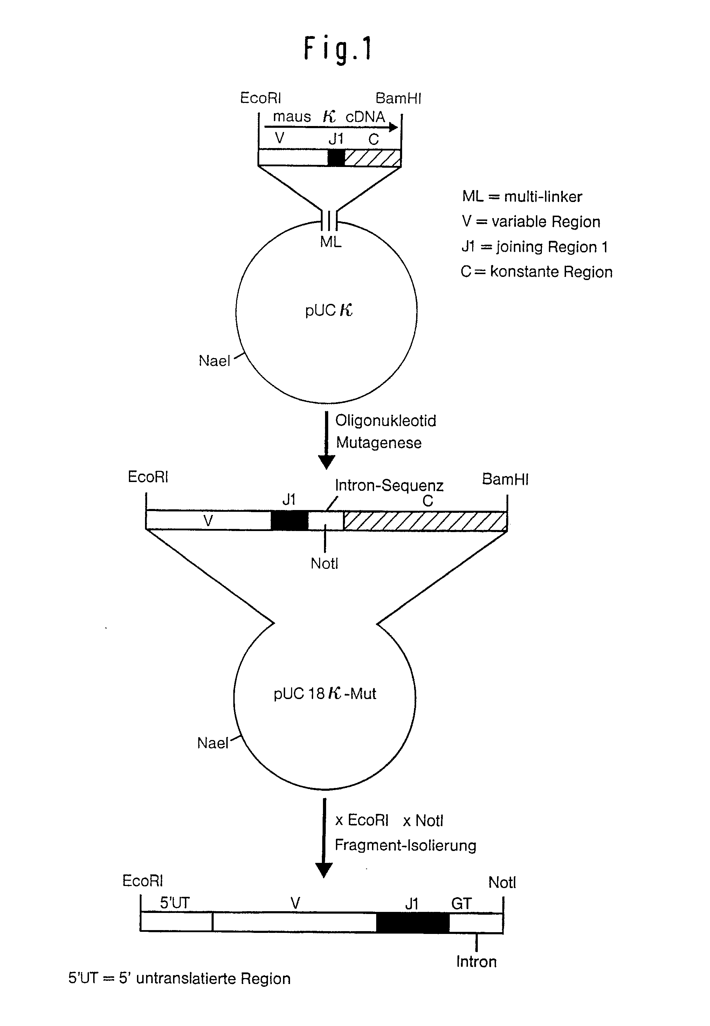

a) Einführung von Spleiß-Donor-, Intron- und NotI-Sequenzen an VJ1 Region der kappa-Kette von MAX179 (Fig. 1).

Die cDNA für die leichte Kette des murinen MAX 179 wurde als EcoRI-HpaI Fragment in pUC18 (Yanisch-Perron, C., et al.: Gene 33 (1985) 103-109) subkloniert, wobei das Plasmid pUCk entstand. Durch Mutagenese wird ein über EcoRI- und NotI-Enden klonierbares (portables) EcoRI-NotI-Fragment erzeugt, welches die VJ1 Region entsprechend der genomischen Organisation der Maus kappa-Ketten enthält. Daran schließen sich an 20 Nukleotide Intron-Sequenzen beginnend mit der Spleiß-Donor-Sequenz GT des genomischen J1 Segmentes der Maus kappa-Kette (Max, E.E., et al., J. Biol. Chem. 256 (1981) 5116-5220) und eine Erkennungs-Sequenz (GCGGCCGC) für die Restriktions-Endonuklease NotI. Die Mutagenese (s.u.) wird durchgeführt nach Morinaga, et al. (Bio/Technology 2 (1984) 636-639). Sequenz des für die Mutagenese verwendeten Oligonukleotides:

<u>Spleiß-Donor-Sequenz</u>

5′GCAAATCAAAC GTAAGTAGAATCCAAAGTCT GCGGCCGC GGGCTGATGCT 3′

J1          J1 Intron          Not I     Maus kappa

konstante

Region

Zur Heteroduplex-Bildung wurden aus pUCk zwei Fragmente isoliert.

Fragment A: pUCk wurde mit den Restriktions-Enzymen EcoRI und BamHI gespalten, die beiden Fragmente gelelektrophoretisch (Agarose-Gel) aufgetrennt und das große EcoRI-BamHI Fragment aus dem Gel isoliert. Dieses Fragment enthält keine Maus kappa Sequenzen.

Fragment B: pUCk wurde mit Nael behandelt (unikate Schnittstelle im pUC-Anteil), die 5'-Phosphat-Reste durch Behandlung mit alkalischer Phosphatase entfernt und anschließend das Fragment zwei mal auf einem 0.7 %igen Agarose-Gel aufgereinigt.

Zur Heteroduplex-Bildung wurden Fragment A; Fragment B (jeweils 500 fmol) und das Oligonukleotid (80 fmol) gemischt und in 50 mmol/l NaCl, 10 mmol/l Tris-HCl, pH 7.5, 10 mmol/l MgSO$_4$ drei Minuten bei 100° C inkubiert und danach auf Eis überführt. Anschließend wurde die DNA renaturiert (20 min bei 60° C). Zur Reparatur-Synthese wurde der Reaktions-Ansatz eingestellt auf: Desoxynukleosidtriphosphate (0.25 mmol/l), ATP (1 mmol/l), NaCl (100 mmol/l), Tris • HCl pH 7.5 (6.5 mmol/l), MgCl$_2$ (8 mmol/l), $\beta$-Mercaptoethanol (1 mmol/l), Klenow-Fragment der DNA-Polymerase aus E.coli (0.125 U/$\mu$l Ansatz) und T4 Ligase (0.1 U/$\mu$l Ansatz) und 4 Stunden bei 16° C inkubiert. Anschließend wurde mit dem Reaktions-Ansatz E.coli HB101 transformiert und Kolonien auf Ampicillinhaltigen (50 $\mu$g/ml) Agar-Platten selektioniert. Mit Hilfe der Koloniehybridisierungstechnik (Maniatis et al.: Molecular Cloning. A Laboratory Manual. Cold Spring Harbor Laboratory, Cold Spring Harbor, NY 1982) konnten diejenigen Kolonien identifiziert werden, in deren Plasmid DNA das oben beschriebene Oligonukleotid eingebaut wurde. Die Charakterisierung erfolgte zunächst durch Analyse mit Restriktions-Endonukleasen. Schließlich wurde die gewünschte. Änderung der Sequenz durch Sequenzierung bestätigt (Sanger, F., et al.: Proc. Natl. Acad. Sci. USA **74** (1977) 5463-5467).

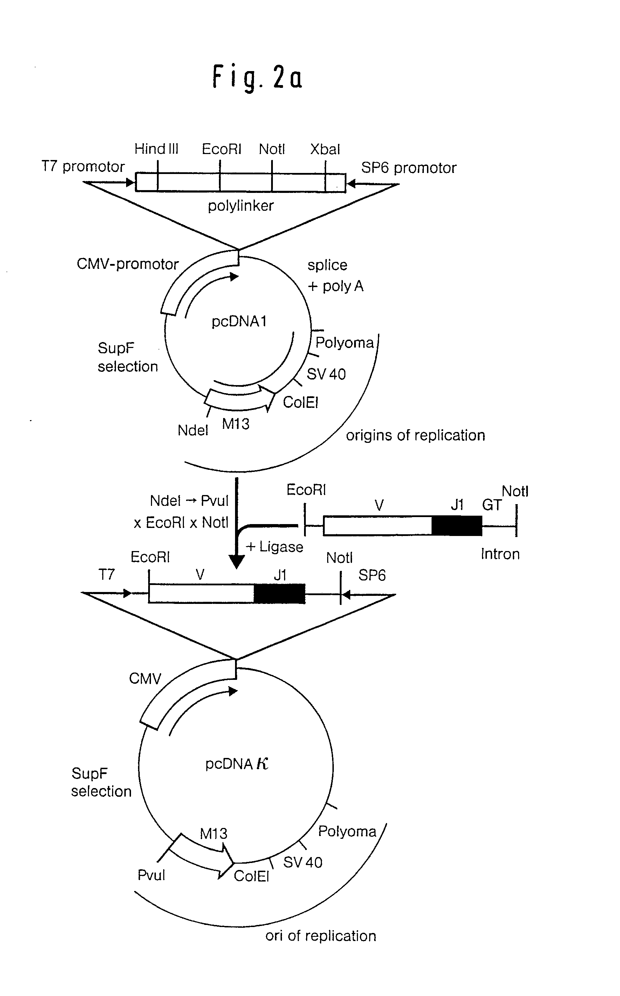

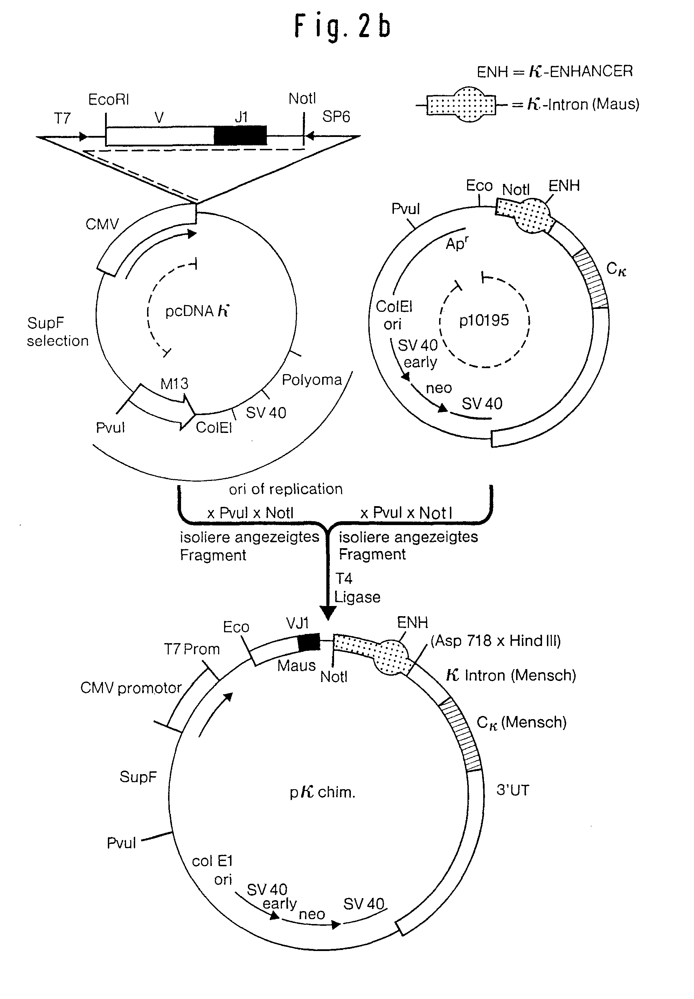

b) Konstruktion eines Expressions-Vektors für die chimärisierte kappa-Kette von MAK 179 (Fig. 2a und 2b).

Als Ausgangs-Plasmid diente pcDNA1 (Invitrogen, San Diego; Aruffo, A. and Seed, B. (1987) Proc. Natl. Acad. Sci. USA **84**, 8573-8577). Es enthält den Promotor des menschlichen Cytomegalie-Virus, Promotoren der Phagen T7 und SP6, einen Polylinker, Spleiß- und Polyadenylierungs-Sequenzen von SV40, zur Selektion eine supF tRNA sowie "origins of replication" von M13, ColE1, SV40 und Polyoma. Aus Klonierungs-Gründen wurde die eingezeichnete Ndel Stelle durch Einligieren eines entsprechenden Linkers in eine Pvul Stelle umgewandelt (= pcDNA1-Pvul). Letzteres Plasmid wurde im Polylinker mit EcoRI und Notl geschnitten und das EcoRI-Notl Fragment der gemäß (a) erhaltenen DNA (ca. 400 bp), enthaltend die VJ1 Region und Intron-Sequenzen von MAK179kappa, in den Vektor einligiert. Der Ligierungs-Ansatz wurde in den E.coli-Stamm MC1061/P3 (Aruffo, A. und Seed, B.: Proc. Natl. Acad. Sci. USA **84** (1987) 8573-8577; Seed, B.: Nucl. Acids Res. **11** (1983) 2427-2445) transfektiert und Ampicillin resistente Kolonien auf Agar-Platten isoliert. Das entstandene Plasmid wird mit pcDNAk bezeichnet. pcDNAk wurde dann mit Pvul und Notl geschnitten und das kürzere Fragment auf einem niedrig-schmelzenden Agarose-Gel isoliert.

Das Plasmid p10195 (Herstellung nach EP-A 0 378 175) wurde ebenfalls mit Notl und Pvul geschnitten und das größere Fragment (Fig. 2b) auf einem niedrig-schmelzenden Agarose-Gel isoliert. p10195 enthält Intron Sequenzen der Maus für die kappa-Kette sowie kodierende und nicht-kodierende Bereiche der konstanten humanen kappa Region.

Die beiden Fragmente von pcDNAk und p10195 wurden mit T4 Ligase behandelt, in MC1061/P3 transfektiert und Ampicillin resistente Kolonien isoliert. Das entstandene Plasmid wird mit pk chim. bezeichnet. Das Hybrid-Gen (VJ1-Maus-C$_{kappa}$-Human) wird unter der Kontrolle des humanen CMV-Promotors (Cytomegalie-Virus) exprimiert. Eine Expressions-Kassette für die Phosphotransferase neo (von p10195 in pk chim. eingebracht) dient der Selektion von G418 resistenten Kolonien nach Transfektion in Säuger-Zellen (Southern, P. and Berg, P.: J. Mol. Appl. Genet. 1 (1982) 327-341).

Beispiel 3

Chimärisierung der schweren Kette von MAK179 und Konstruktion eines Expressions-Vektors für die chimärisierte schwere Kette

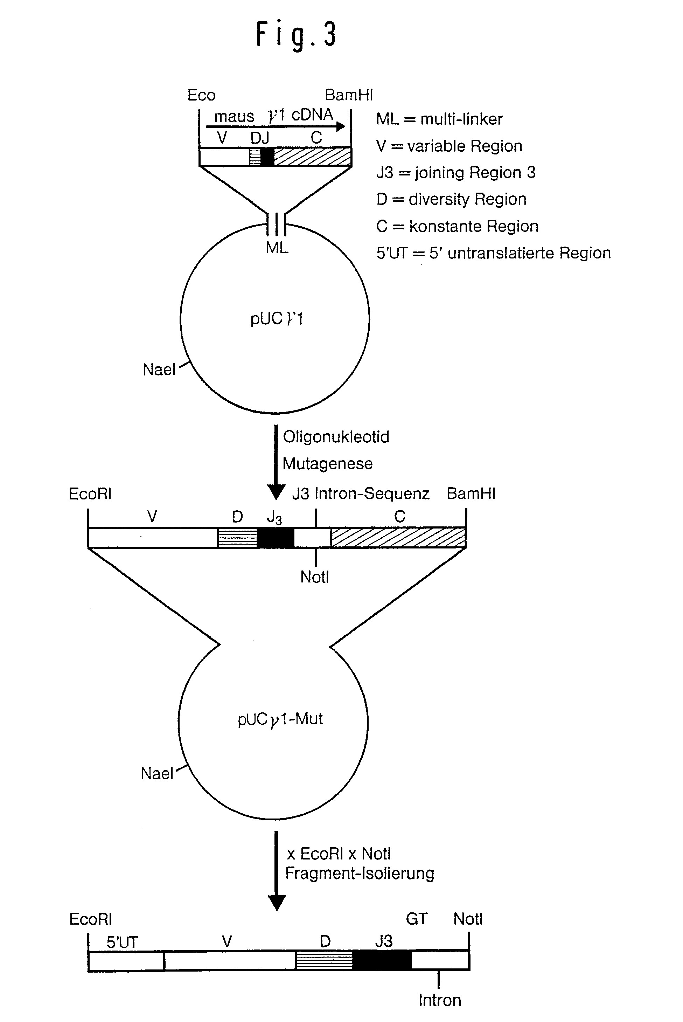

a) Einführung von Spleiß-Donor, Intron und Notl Sequenzen in die VDJ$_3$-Region der $\gamma$1 Kette von MAK179 (Fig. 3).

Die cDNA für die $\gamma$1-Kette (5'untranslatierte Region und codierende Region bis zur BamHI-Stelle in der konstanten Region) wurde als EcoRI-BamHI Fragment in pUC18 (Yanisch-Perron, C., et al.: Gene 33 (1985) 103-109) subkloniert = Plasmid pUC$\gamma$1.

Durch Mutagenese wird ein portables EcoRI-Notl Fragment erzeugt, welches die VDJ3 Region entsprechend der genomischen Organisation der Maus $\gamma$1-Kette, sich daran anschließend 20 Nukleotide Intron-Sequenzen beginnend mit der Spleiß-Donor-Sequenz GT des genomischen J3 Segmentes der Maus $\gamma$1-Kette (Sakano, et al., Nature 286 (1980) 676-682) und sich daran anschließend eine Erkennungs-Sequenz (GCGGCCGC) für die Restriktions-Endonuklease Notl enthält. Die Mutagenisierung wurde durchgeführt

7

nach Morinaga, et al. (Bio/Technology 2 (1984) 636-639). Sequenz des für die Mutagenese verwendeten Oligonukleotides:

## Spleiß Donor Sequenz

5'GTCTCTGCAG  GTGAGTCCTAACTTCTCCCA  GCGGCCGC  TGCCTGGTCA3'

J3           J3 Intron             NotI      Maus $\gamma$1

konstante Region

Zur Heteroduplex-Bildung wurden aus pUC$\gamma$1 zwei Fragmente isoliert.

Fragment C: pUC$\gamma$1 wurde mit den Restriktions-Enzymen EcoRI und BamHI gespalten, die beiden Fragmente gelelektrophoretisch (1 %iges Agarose-Gel) aufgetrennt und das große EcoRI-BamHI Fragment aus dem Gel isoliert. Dieses Fragment enthält keine Maus $\gamma$1-Sequenzen.

Fragment D: pUC$\gamma$1 wurde mit NaeI geschnitten (unikate Schnittstelle im pUC- Anteil), die 5'-Phosphat-Reste durch Behandlung mit alkalischer Phosphatase entfernt und anschließend das Fragment zwei mal auf einem 0.7 %igen Agarose-Gel aufgereinigt. Nach Mischung der Fragmente C und D (jeweils 500 fmol) und des Oligonukeotids (80 fmol) wurde die Mutagenese wie in Beispiel 2 beschrieben durchgeführt. Mit Hilfe der Kolonie-Hybridisierungs-Technik (Maniatis, et al: Molecular Cloning. A Laboratory Manual. Cold Spring Harbor Laboratory, Cold Spring Harbor, NY 1982) konnten diejenigen Kolonien identifiziert werden, in deren Plasmid-DNA das oben beschriebene Oligonucleotid eingebaut wurde. Die Charakterisierung erfolgte zunächst mit Restriktions-Endonukleasen. Schließlich wurde die gewünschte Änderung der Sequenz durch Sequenzierung bestätigt (Sanger, F., et al.: Proc. Natl. Acad. Sci. USA 74 (1977) 5463-5467). Fig. 3 zeigt schematisch die Herstellung des gewünschten EcoRI- NotI Fragments.

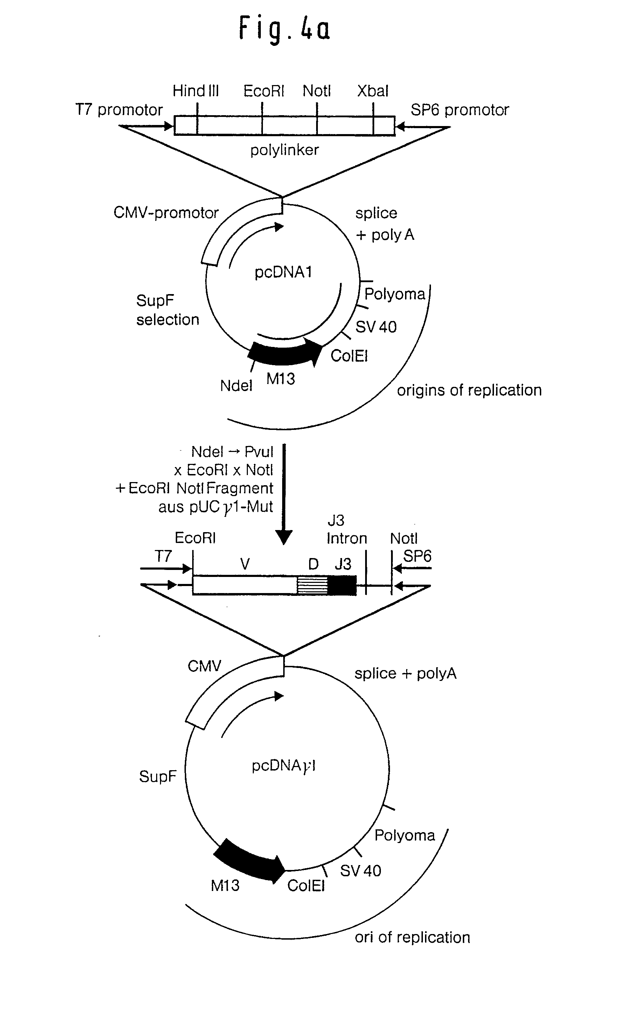

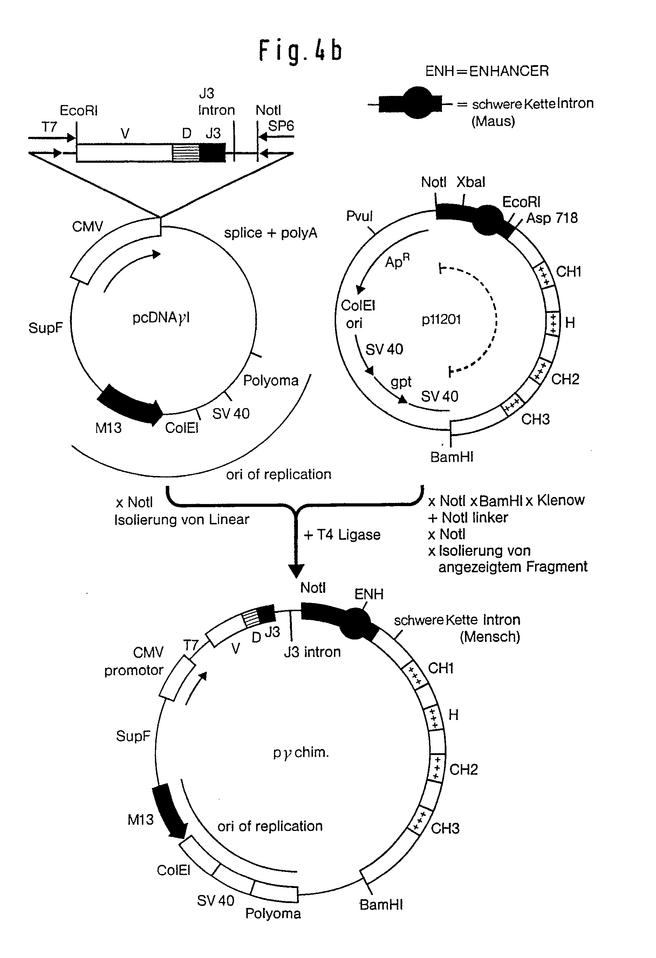

b) Konstruktion von Expressions-Vektor für chimärisierte $\gamma$1 Kette von MAK179 (Fig. 4a und 4b). Die unikate NdeI-Stelle im Vektor pcDNA1 (Invitrogen, San Diego; Aruffo, A. and Seed, B. (1987) Proc. Natl. Acad. Sci. USA 84, 8573-8577) wurde durch Einsetzen eines entsprechenden Linkers in eine PvuI-Stelle umgewandelt. Das entstandene Plasmid wird als pcDNA1-PvuI bezeichnet.

pcDNA1-PvuI wurde mit EcoRI und NotI gespalten und das in Abb. 3 beschriebene EcoRI-NotI Fragment von pUC$\gamma$1-Mut (mit VDJ3 und Intron-Sequenzen von $\gamma$1) einligiert. Das entstandene Plasmid wird als pcDNA$\gamma$1 bezeichnet.

Das Plasmid p11201 (Herstellung nach EP-A 0 378 175) wird mit NotI und BamHI gespalten, die überhängenden Enden mit Klenow aufgefüllt, NotI-Linker anligiert, mit NotI nachgespalten und das in Abb. 4b gekennzeichnete Fragment auf einem niedrig-schmelzenden Agarose-Gel isoliert. Dieses Fragment enthält Intron Sequenzen der schweren Ketten der Maus, Intron-Sequenzen der schweren Ketten des Menschen sowie das genomische Äquivalent der konstanten Region des humanen $\gamma$1 Gens. Dieses Fragment wurde in die unikate NotI-Stelle des Vektors pcDNA$\gamma$1 einligiert. Die richtige Orientierung wurde durch Spaltung mit Restriktions-Endonukleasen ermittelt. Der entstandene Expressions-Vektor für die chimärisierte $\gamma$1 Kette von MAK179 wird mit p$\gamma$ chim. bezeichnet. Nach diesem Prinzip können chimärisierte Ketten auch anderer Isotypen (wie z.B. $\mu$, $\gamma$2, $\gamma$3, $\gamma$4, $\alpha$1, $\alpha$2, $\delta$, $\epsilon$) konstruiert werden.

Beispiel 4:

Etablierung permanenter Zell-Linien von Non-producer Hybridom-Linien durch Elektroporation mit Expressions-Plasmiden für die chimärisierten Ketten von MAK179.

Die Plasmide pK chim. und p$\gamma$1 chim. wurden zu gleichen Mengen gemischt und durch Elektroporation (Gene-Pulser von BioRad) in die Immunoglobulin Non-Producer Hybridom Linie Sp2/0-Ag14 (ATCC CRL 8923) (Ochi, A., et al., Proc. Natl. Acad. Sci. USA 80 (1983) 6351-6355) transfektiert. Ebenso geeignet sind auch alle anderen Non-Immunglobulin-Producer Hybridomzellinien wie z.B. P3X63-Ag8.653 (ATCC CRL 8375). Beide Plasmide wurden vor der Transfektion mit der Restriktions-Endonuklease PvuI linearisiert. Die Durchführung der Elektroporation ist in Nucleic Acids Res. 15 (1987) 1311-1326 bzw. Bio Techniques 6 (1988) 742-751 beschrieben. Nach Abzentrifugieren wurden die Zellen mit kaltem HeBS-Puffer gewaschen (20 mmol/l HEPES, pH 7.05, 137 mmol/l NaCl, 5 mmol/l KCl, 0.7 mmol/l Na$_2$HPO$_4$, 6 mmol/l Dextrose), mit HeBS-Puffer resuspendiert, auf eine Konzentration von 10$^6$ Zellen/ml eingestellt und auf Eis gestellt. Nach

EP 0 460 674 A2

Plasmid Zugabe wurde gepulst (Bedingungen: Kapazität 500 μF und Spannungs-Bereich zwischen 240 und 280 V oder bei 160 μF und 200 bis 240 V). Die Zellen wurden nach dem Pulsen ca. 10 Minuten auf Eis gehalten und anschließend in Medium I (RPMI 1640, 10 % foetales Kälberserum, 2 mmol/l Glutamin, 1 mmol/l Natriumpyruvat, 0.1 mmol/l nicht-essentielle Aminosäuren) bei 37 °C inkubiert. 30 h nach der Transfektion wurde das Medium gewechselt und mit Medium I + 800 μg/ml G418 inkubiert, nachdem etwa $10^3$ Zellen pro Vertiefung von 96 well Mikrotiter-Platten ausgesät worden waren. Es wurden insgesamt 10 Mikrotiter-Platten (je 96 Vertiefungen) angelegt. 7-10 Tage nach der Aussaat konnten in den Vertiefungen G418 resistente Kolonien identifiziert werden. Ihre Kulturüberstände wurden wie im folgenden Beispiel beschrieben auf rekonstituierten chimärisierten MAK179 getestet. Die besten Producer wurden in Massenkultur in Medium I vermehrt.

Beispiel 5:

Bestimmung von rekonstituiertem Antikörper gegen den humanen IL-2-Rezeptor

Zunächst wird eine Mikrotiterplatte mit polyklonalen Antikörpern gegen humanes Fcγ beschichtet. Dazu werden die Vertiefungen einer Mikrotiter-Platte mit 200 μl entsprechend 2.5 μg eines polyklonalen Antikörpers gegen humanes Fcγ (IgG) in 0.2 mol/l Carbonat/Bicarbonat, pH 9.5 über Nacht bei 4 °C oder 1 Stunde bei Raumtemperatur inkubiert. Nach Aussaugen der Vertiefungen wurde mit 300 μl 50 mmol/l HEPES, 0.15 mol/l NaCl/1 % Crotein C, pH 7.0 30 min bis 1 h bei Raumtemperatur inkubiert. Anschließend wurden 200 μl Eichprobe oder Kulturüberstände von transfektierten Zellen zugegeben und 1 h bei Raumtemperatur unter Schütteln (500 rpm) oder 90 min bei Raumtemperatur ohne Schütteln inkubiert. Die Eichkurve wurde mit einem Modell-Chimär erstellt (hergestellt durch chemische Verbrückung von einem humanen IgG MAK und Fab-Fragmenten von MAK179). Nach Aussaugen der Vertiefungen wurde 2 mal gewaschen mit je 300 μl IP = Inkubations-Puffer (50 mmol/l HEPES, pH 7.0, 0.15 mol/l NaCl, 0.2 mol/l Di-Natrium-tartrat, 1 % Crotein C, 0,75 % PEG 40.000 (Serva), 0.5 % Pluronic F68 (Boehringer Mannheim), 0.01 % Phenol). Anschließend wurden 200 μl IL-2-Rezeptor-POD-Antikörper-Konjugatkomplex-Lösung (s.u.) zugegeben und 1 h bei Raumtemperatur unter Schütteln (500 rpm) oder 90 min bei Raumtemperatur ohne Schütteln inkubiert. Der vorgebildete Komplex besteht aus löslichem Interleukin-2-Rezeptor und POD markierten Fab-Fragmenten von IL-2-Rezeptor MAK215.

Herstellung der Komplex-Lösung: 20 μl der POD-markierten Fab-Fragmente von MAK 215 (150 U/ml) + 19.5 ml Inkubations-Puffer IP (s.o.) + 3 ml löslicher IL-2-Rezeptor Standard (6400 U/ml; Units definiert über Standard von T-cell Sciences). Der lösliche IL-2-Rezeptor wurde gewonnen aus Kulturüberständen der von Shimizu, et al. (Mol. Biol. Med. 3 (1986) 509-520) beschriebenen Maus-Fibroblasten Zell-Linie. Nach Durchführung der Inkubation wurde 3 mal mit dem oben beschriebenen Wasch-Puffer gewaschen und anschließend die POD Aktivität mit ABTS-Lösung [2,2'-azino-di-3-ethylbenzthiazo-linsulfonat] + $H_2O_2$ nach 45-60 min Inkubation bei Raumtemperatur durch Ablesen der Extinktion bei 405 nm in einem ELISA-Reader bestimmt. Es wurden mit dieser Methode Klone mit hoher Expression (bis zu 10 μg/ml) bestimmt.

9

SEQ ID NO: 1    (Leichte Kette Klon 179)

ART DER SEQUENZ: Nucleotid mit entsprechendem Protein
SEQUENZLAENGE: 432 Basenpaare

MERKMALE:        aa -20 (Met) : Start der Signalsequenz
                 aa   1 (Asp) : Beginn der V-Region
          von aa  96 (Arg) bis aa 107 (Lys) J1-Region
          ab  aa 108 (Arg) : Beginn der C-Region


```
ATG ATG GTC CTT GCT CAG TTT CTT GCA TTC TTG TTG CTT TGG TTT CCA   48
Met Met Val Leu Ala Gln Phe Leu Ala Phe Leu Leu Leu Trp Phe Pro
-20             -15             -10                         -5

GGT GCA AGA TGT GAC ATC CTG ATG ACC CAA TCT CCA TCC TCC ATG TCT   96
Gly Ala Arg Cys Asp Ile Leu Met Thr Gln Ser Pro Ser Ser Met Ser
                1               5                       10

GTA TCT CTG GGA GAC ACA GTC AGC ATC ACT TGC CAT GCA AGT CAG GGC   144
Val Ser Leu Gly Asp Thr Val Ser Ile Thr Cys His Ala Ser Gln Gly
        15                  20                  25

ATC AGA AGT AAT ATA GTG TGG TTG CAG CAG AAA CCA GGG AAA TCA TTT   192
Ile Arg Ser Asn Ile Val Trp Leu Gln Gln Lys Pro Gly Lys Ser Phe
        30                  35                  40

AGG GGC CTG ATC TAT CAT GGA ACC AAG TTG GAA GAT GGA GTT CCA TCA   240
Arg Gly Leu Ile Tyr His Gly Thr Lys Leu Glu Asp Gly Val Pro Ser
45                  50                  55                  60

AGG TTC AGT GGC AGT GGA TCT GGA GCA GAT TAT TCT CTC ACC ATC AGC   288
Arg Phe Ser Gly Ser Gly Ser Gly Ala Asp Tyr Ser Leu Thr Ile Ser
                65                  70                  75

AGC CTG GAA TCT GAA GAT TTT GCA GAC TAT TAT TGT GTA CAG TAT GCT   336
Ser Leu Glu Ser Glu Asp Phe Ala Asp Tyr Tyr Cys Val Gln Tyr Ala
                80                  85                  90

CAG TTT CCT CGG ACG TTC GGT GGA GGC ACC AAG CTG GAA ATC AAA CGG   384
Gln Phe Pro Arg Thr Phe Gly Gly Gly Thr Lys Leu Glu Ile Lys Arg
            95                  100                 105

GCT GAT GCT GCA CCA ACT GTA TCC ATC TTC CCA CCA TCC AGT GAG CAG   432
Ala Asp Ala Ala Pro Thr Val Ser Ile Phe Pro Pro Ser Ser Glu Gln
    110                 115                 120
```

SEQ ID NO: 2    (Leichte Kette Klon 447)

ART DER SEQUENZ: Nucleotid mit entsprechendem Protein
SEQUENZLAENGE: 432 Basenpaare

MERKMALE:        aa -20 (Met) : Start der Signalsequenz
                 aa   1 (Asp) : Beginn der V-Region
           von aa  96 (Arg) bis aa 107 (Lys) J1-Region
           ab  aa 108 (Arg) : Beginn der C-Region


```
ATG ATG GTC CTT GCT CAG TTT CTT GCA TTC TTG TTG CTT TGG TTT CCA   48
Met Met Val Leu Ala Gln Phe Leu Ala Phe Leu Leu Leu Trp Phe Pro
-20             -15             -10                         -5

GGT GCA AGA TGT GAC ATC CTG ATG ACC CAA TCT CCA TCC TCC ATG TCT   96
Gly Ala Arg Cys Asp Ile Leu Met Thr Gln Ser Pro Ser Ser Met Ser
                 1               5                   10

GTT TCT CTG GGA GAC ACA GTC ACC ATC ACT TGC CAT GCA AGT CAG GGC  144
Val Ser Leu Gly Asp Thr Val Thr Ile Thr Cys His Ala Ser Gln Gly
        15              20              25

ATT AGA AGT AAT ATA GTG TGG TTG CAG CAG AAA CCA GGG AAA TCA TTT  192
Ile Arg Ser Asn Ile Val Trp Leu Gln Gln Lys Pro Gly Lys Ser Phe
    30              35              40

AAG GGC CTG ATC TAT CAT GGA ACC AAC TTG GAA GAT GGA GTT CCA TCA  240
Lys Gly Leu Ile Tyr His Gly Thr Asn Leu Glu Asp Gly Val Pro Ser
45              50              55                      60

CGG TTC AGT GGC AGT GGA TCT GGA GCA GAT TAT TCT CTC ACC ATC AGC  288
Arg Phe Ser Gly Ser Gly Ser Gly Ala Asp Tyr Ser Leu Thr Ile Ser
                65              70                      75

AGC CTG GAA TCT GAA GAT TTT GCA GAC TAT TAC TGT GTA CAG TAT GCT  336
Ser Leu Glu Ser Glu Asp Phe Ala Asp Tyr Tyr Cys Val Gln Tyr Ala
            80              85              90

CAG TTT CCT CGG ACG TTC GGT GGA GGC ACC AAG CTG GAA ATC AAA CGG  384
Gln Phe Pro Arg Thr Phe Gly Gly Gly Thr Lys Leu Glu Ile Lys Arg
        95              100             105

GCT GAT GCT GCA CCA ACT GTA TCC ATC TTC CCA CCA TCC AGT GAG CAG  432
Ala Asp Ala Ala Pro Thr Val Ser Ile Phe Pro Pro Ser Ser Glu Gln
    110             115             120
```

SEQ ID NO: 3    (Leichte Kette Klon 215)

ART DER SEQUENZ: Nucleotid mit entsprechendem Protein
SEQUENZLAENGE: 435 Basenpaare

MERKMALE:         aa  -22 (Met) : Start der Signalsequenz
                  aa   1 (Lys) : Beginn der V-Region
              von aa  95 (Phe) bis aa 106 (Lys) J4-Region
              ab  aa 107 (Arg) : Beginn der C-Region


ATG GAT TTT CAA GTG CAG ATT TTC AGC TTC CTG CTA ATC AGT GCT TCA  48
Met Asp Phe Gln Val Gln Ile Phe Ser Phe Leu Leu Ile Ser Ala Ser
        -20              -15              -10

GTC ATA ATG TCC AGA GGC AAA ATT GTT CTC TCC CAG TCT CCA GCA ATC  96
Val Ile Met Ser Arg Gly Lys Ile Val Leu Ser Gln Ser Pro Ala Ile
        -5               1               5                   10

CTG TCT GCA TCT CCA GGG GAG AAG GTC ACA ATG ACT TGC AGG GCC AGC  144
Leu Ser Ala Ser Pro Gly Glu Lys Val Thr Met Thr Cys Arg Ala Ser
                15              20                  25

TCA AGT ATA AGT TAC ATG CAC TGG TAC CAG CAG AAG CCA GGA TCC TCC  192
Ser Ser Ile Ser Tyr Met His Trp Tyr Gln Gln Lys Pro Gly Ser Ser
            30              35                  40

CCC AAA CCC TGG ATT CAA GCC ACA TCC AAC CTG GCT TTT GGA GTC CCT  240
Pro Lys Pro Trp Ile Gln Ala Thr Ser Asn Leu Ala Phe Gly Val Pro
            45              50                  55

TCT CGC TTC AGT GGC AGT GGG TCT GGG ACC TCT TAC TCT CTC ACA ATC  288
Ser Arg Phe Ser Gly Ser Gly Ser Gly Thr Ser Tyr Ser Leu Thr Ile
        60              65                  70

AGC AGA GTG GAG GCT GAA GAT GCT GCC ACT TAT TAC TGC CAG CAG TGG  336
Ser Arg Val Glu Ala Glu Asp Ala Ala Thr Tyr Tyr Cys Gln Gln Trp
    75              80                  85                  90

AGT AGT AAC CCA TTC ACG TTC GGC TCG GGG ACA AAG TTG GAA ATG AAA  384
Ser Ser Asn Pro Phe Thr Phe Gly Ser Gly Thr Lys Leu Glu Met Lys
                95              100                 105

CGG GCT GAT GCT GCA CCA ACT GTA TCC ATC TTC CCA CCA TCC AGT GAG  432
Arg Ala Asp Ala Ala Pro Thr Val Ser Ile Phe Pro Pro Ser Ser Glu
        110                 115                 120

CAG   435
Gln

SEQ ID NO: 4    (Schwere Kette Klon 179)

ART DER SEQUENZ: Nucleotid mit entsprechendem Protein
SEQUENZLAENGE: 531 Basenpaare

MERKMALE:        aa  -19 (Met) : Start der Signalsequenz
                 aa   1 (Asp) : Beginn der V-Region
          von aa  99 (Asp) bis aa 102 (Asn)  D-Region
          von aa 103 (Trp) bis aa 113 (Ala) J3-Region
          ab  aa 114 (Ala) : Beginn der C-Region


ATG GAC TCC AGG CTC AAT TTA GTT TTC CTT GTC CTT ATT TTA AAA GGT    48
Met Asp Ser Arg Leu Asn Leu Val Phe Leu Val Leu Ile Leu Lys Gly
            -15             -10                     -5

GTC CAG TGT GAT GTG CAG CTG GTG GAG TCT GGG GGA GGC TTA GTG CAG    96
Val Gln Cys Asp Val Gln Leu Val Glu Ser Gly Gly Gly Leu Val Gln
             1               5                   10

CCT GGA GGG TCC CGG AAA CTC TCC TGT GTT GCC TCT GGA TTC ACT TTC   144
Pro Gly Gly Ser Arg Lys Leu Ser Cys Val Ala Ser Gly Phe Thr Phe
        15                  20                  25

AGT ACC TTT GGA ATG CAC TGG GTT CGT CAG GCT CCA GAG AAG GGG CTG   192
Ser Thr Phe Gly Met His Trp Val Arg Gln Ala Pro Glu Lys Gly Leu
 30                  35                  40                  45

GAG TGG GTC GCA TAC ATT AGT AGT GGC AGT GGT ACC ATC TAC TAT GCA   240
Glu Trp Val Ala Tyr Ile Ser Ser Gly Ser Gly Thr Ile Tyr Tyr Ala
                 50                  55                  60

GAC ACA GTG AAG GGC CGA TTC ACC ATC TCC AGA GAC AAT CCC AAG AAT   288
Asp Thr Val Lys Gly Arg Phe Thr Ile Ser Arg Asp Asn Pro Lys Asn
             65                  70                  75

ACC CTG TTC CTG CAA ATG ACC AGT CTA AGG TCT GAG GAC ACG GCC ATG   336
Thr Leu Phe Leu Gln Met Thr Ser Leu Arg Ser Glu Asp Thr Ala Met
             80                  85                  90

TAT TAC TGT GCA AGA GAT TGG ATG AAC TGG GGC CAA GGG ACT CTG GTC   384
Tyr Tyr Cys Ala Arg Asp Trp Met Asn Trp Gly Gln Gly Thr Leu Val
     95                  100                 105

ACT GTC TCT GCA GCC AAA ACG ACA CCC CCA TCT GTC TAT CCA CTG GCC   432
Thr Val Ser Ala Ala Lys Thr Thr Pro Pro Ser Val Tyr Pro Leu Ala
110                 115                 120                 125

CCT GGA TCT GCT GCC CAA ACT AAC TCC ATG GTG ACC CTG GGA TGC CTG   480
Pro Gly Ser Ala Ala Gln Thr Asn Ser Met Val Thr Leu Gly Cys Leu
                130                 135                 140

GTC AAG GGC TAT TTC CCT GAG CCA GTG ACA GTG ACC TGG AAC TCT GGA   528
Val Lys Gly Tyr Phe Pro Glu Pro Val Thr Val Thr Trp Asn Ser Gly
                145                 150                 155

TCC    531
Ser

SEQ ID NO: 5    (Schwere Kette Klon 447)

ART DER SEQUENZ: Nucleotid mit entsprechendem Protein
SEQUENZLAENGE: 531 Basenpaare

MERKMALE:      aa  -19 (Met) : Start der Signalsequenz
               aa    1 (Asp) : Beginn der V-Region
         von aa   99 (Asp) bis aa 102 (Asn)  D-Region
         von aa  103 (Trp) bis aa 113 (Thr) J3-Region
         ab   aa  114 (Ala) : Beginn der C-Region


```
ATG GAC TCC AGG CTC AAT TTA GTG TTC CTT GTC CTT ATT TTA AAA GGT   48
Met Asp Ser Arg Leu Asn Leu Val Phe Leu Val Leu Ile Leu Lys Gly
            -15                 -10                 -5

GTC CAG TGT GAT GTG CAA CTG GTG GAG TCT GGG GGA GGC TTA GTG CAG   96
Val Gln Cys Asp Val Gln Leu Val Glu Ser Gly Gly Gly Leu Val Gln
            1               5                   10

CCT GGA GGG TCC CGG AAA CTC TCC TGT GCA GCC TCT GGA TTC ACT TTC  144
Pro Gly Gly Ser Arg Lys Leu Ser Cys Ala Ala Ser Gly Phe Thr Phe
        15              20              25

AGT ACC TTT GGA ATG CAC TGG GTT CGT CAG GCT CCA GAG AAG GGG CTG  192
Ser Thr Phe Gly Met His Trp Val Arg Gln Ala Pro Glu Lys Gly Leu
    30              35              40                  45

GAG TGG GTC GCA TAT ATT AGT AGT GGC AGT AGT ACC GTC TAC TAT GCA  240
Glu Trp Val Ala Tyr Ile Ser Ser Gly Ser Ser Thr Val Tyr Tyr Ala
                50              55                  60

GAC AGA GTG AGG GGC CGA TTC ACC ATC TCC AGA GAC AAT CCC AAG AAC  288
Asp Arg Val Arg Gly Arg Phe Thr Ile Ser Arg Asp Asn Pro Lys Asn
            65              70              75

ACC CTG TTC CTG GAA ATG ACC AGT CTA AGG TCT GAG GAC ACG GCC ATG  336
Thr Leu Phe Leu Glu Met Thr Ser Leu Arg Ser Glu Asp Thr Ala Met
            80              85              90

TAT TAC TGT GCA AGA GAT TGG ATG AAC TGG GGC CAA GGG ACT CTG GTC  384
Tyr Tyr Cys Ala Arg Asp Trp Met Asn Trp Gly Gln Gly Thr Leu Val
    95              100             105

ACT GTC TCT ACA GCC AAA ACG ACA CCC CCA TCT GTC TAT CCA CTG GCC  432
Thr Val Ser Thr Ala Lys Thr Thr Pro Pro Ser Val Tyr Pro Leu Ala
110             115             120                 125

CCT GGA TCT GCT GCC CAA ACT AAC TCC ATG GTG ACC CTG GGA TGC CTG  480
Pro Gly Ser Ala Ala Gln Thr Asn Ser Met Val Thr Leu Gly Cys Leu
            130             135             140

GTC AAG GGC TAT TTC CCT GAG CCA GTG ACA GTG ACC TGG AAC TCT GGA  528
Val Lys Gly Tyr Phe Pro Glu Pro Val Thr Val Thr Trp Asn Ser Gly
            145             150             155

TCC    531
Ser
```

SEQ ID NO: 6    (Schwere Kette Klon 215)

ART DER SEQUENZ: Nucleotid mit entsprechendem Protein
SEQUENZLAENGE: 549 Basenpaare

MERKMALE:        aa -19 (Met) : Start der Signalseuqenz
                 aa   1 (Gln) : Beginn der V-Region
          von aa  98 (Thr) bis aa 104 (Ser)  D-Region
          von aa 105 (Trp) bis aa 119 (Ala) J3-Region
          ab  aa 120 (Ala) : Beginn der C-Region


ATG GCT GTG CTG GGG CTG CTT CTC TGC CTG GTG ACT TTC CCA AGC TGT    48
Met Ala Val Leu Gly Leu Leu Leu Cys Leu Val Thr Phe Pro Ser Cys
            -15               -10                       -5

GTC CCG TCC CAG GTG CAG CTG AAG GAG TCA GGG CCT GGC CTG GTG GCG    96
Val Pro Ser Gln Val Gln Leu Lys Glu Ser Gly Pro Gly Leu Val Ala
            1               5                       10

CCC TCA CAG AGC CTG TCC ATC ACA TGC ACC GTC TCA GGG TTC TCA TTA   144
Pro Ser Gln Ser Leu Ser Ile Thr Cys Thr Val Ser Gly Phe Ser Leu
        15                  20                  25

AGT ACC TAT AGT GTA TAC TGG GTT CGC CAG CCT CCA GGA AAG GGT CTG   192
Ser Thr Tyr Ser Val Tyr Trp Val Arg Gln Pro Pro Gly Lys Gly Leu
 30              35                  40                      45

GAG TGG CTG GGA GTG ATA TGG AGT GAT GGA AGC ACA ACC TAT AAT TCA   240
Glu Trp Leu Gly Val Ile Trp Ser Asp Gly Ser Thr Thr Tyr Asn Ser
                50                  55                  60

ACT CTC AAA TCC AGA CTG ACC ATC AGC AAG GAC AAC TCC AAG AGT CAA   288
Thr Leu Lys Ser Arg Leu Thr Ile Ser Lys Asp Asn Ser Lys Ser Gln
                65                  70                  75

GTT TTC TTA AAA GTG AAC AGT CTC CAA ACT GAT GAC ACA GCC ATG TAC   336
Val Phe Leu Lys Val Asn Ser Leu Gln Thr Asp Asp Thr Ala Met Tyr
            80                  85                  90

TAC TGT GCC AGA ACC TAT GGT TAT GAC GGG TCC TGG CTT GCT TAC TGG   384
Tyr Cys Ala Arg Thr Tyr Gly Tyr Asp Gly Ser Trp Leu Ala Tyr Trp
     95                  100                 105

GGC CAA GGG ACT CTG GTC ACT GTC TCT GCA GCC AAA ACA ACA CCC CCA   432
Gly Gln Gly Thr Leu Val Thr Val Ser Ala Ala Lys Thr Thr Pro Pro
 110             115                 120                     125

TCA GTC TAT CCA CTG GCC CCT GGG TGT GGA GAT ACA ACT GGT TCC TCC   480
Ser Val Tyr Pro Leu Ala Pro Gly Cys Gly Asp Thr Thr Gly Ser Ser
                130                 135                 140

GTG ACT CTG GGA TGC CTG GTC AAG GGC TAC TTC CCT GAG TCA GTG ACT   528
Val Thr Leu Gly Cys Leu Val Lys Gly Tyr Phe Pro Glu Ser Val Thr
            145                 150                 155

GTG ACT TGG AAC TCT GGA TCC    549
Val Thr Trp Asn Ser Gly Ser
            160

**Patentansprüche**

**1.** Rekombinante DNA, welche für die leichte oder schwere Kette eines Antikörpers kodiert, dessen konstante Regionen humanen Ursprungs sind und dessen variable Regionen nicht humanen Ursprungs sind,

**dadurch gekennzeichnet,**

daß sie in Richtung der Transkription

(a) eine für die variable nicht-humane Region kodierende cDNA-Sequenz, einschließlich der Region J und gegebenenfalls D,

(b) eine Intron-Sequenz, welche an ihrem 5'-Ende eine Splice-Donor-Stelle aufweist und zusammengesetzt ist aus einer nicht-humanen Intron-Teilsequenz am 5'-Ende und einer humanen Intron-Teilsequenz am 3'-Ende, und

(c) eine für die humane konstante Region kodierende genomische DNA-Sequenz aufweist.

**2.** Rekombinante DNA nach Anspruch 1,
**dadurch gekennzeichnet,**
daß die Sequenz (a) und die nicht-humane Intron-Teilsequenz von (b) murinen Ursprungs sind.

**3.** Rekombinante DNA nach Anspruch 1 oder 2,
**dadurch gekennzeichnet,**
daß die nicht-humane Intron-Teilsequenz von (b) der auf die Sequenz (a) natürlicherweise folgenden Intron-Sequenz entspricht.

**4.** Rekombinante DNA nach Anspruch 1 oder 2,
**dadurch gekennzeichnet,**
daß die nicht-humane Intron-Teilsequenz von (b) 15 bis 20 Basenpaare lang ist.

**5.** Rekombinante DNA nach einem der vorhergehenden Ansprüche,
**dadurch gekennzeichnet,**
daß die Splice-Donorstelle in (b) die Sequenz 5'-GT-3' aufweist.

**6.** Rekombinante DNA nach einem der vorhergehenden Ansprüche,
**dadurch gekennzeichnet,**
daß die Sequenz (a) für die variable Region der leichten oder schweren Kette eines mit dem humanen Interleukin 2-Rezeptor spezifisch bindefähigen Antikörpers kodiert.

**7.** Rekombinante DNA nach einem der vorhergehenden Ansprüche,
**dadurch gekennzeichnet,**
daß die Sequenz (c) für die konstante Region der schweren Kette eines humanen Antikörpers vom Typ IgG kodiert.

**8.** Expressionsvektor, enthaltend eine rekombinante DNA nach einem der Ansprüche 1 bis 7.

**9.** Plasmid pK chim.

**10.** Plasmid p$\gamma$ chim.

**11.** Verfahren zur Herstellung einer rekombinanten DNA nach einem der Ansprüche 1 bis 7,
**dadurch gekennzeichnet,**
daß man aus einer einen Antikörper gewünschter Spezifität sezernierenden Hybridomzellinie polyA$^+$-mRNA isoliert, eine cDNA-Bibliothek dazu in geeigneten Vektoren herstellt und über Hybridisierung mit zu für den konstanten Teil des Antikörpers kodierender DNA komplementären Oligonukleotiden auf Klone untersucht, welche die cDNA für Antikörperketten enthalten, hieraus die V-, J- und gegebenenfalls D- Sequenzen isoliert, indem man durch gerichtete Mutagenese anschließend an die jeweilige J-Domäne in Transkriptionsrichtung eine Splice-Donor-Stelle, einen Teil einer Antikörper-Intronsequenz nicht humanen Ursprungs und eine Restriktionsschnittstelle einführt und durch Verdauung mit dem entsprechenden Restriktionsenzym von den für die konstante Region kodierenden Sequenzen abtrennt, und die erhaltene DNA mit einer genomischen DNA-Sequenz ligiert, die für die konstante Region der leichten oder schweren Kette eines humanen Antikörpers kodiert und an ihrem 5'-Ende einen Teil einer entsprechenden humanen Intronsequenz aufweist.

12. Verfahren nach Anspruch 11,
**dadurch gekennzeichnet,**
daß man eine Maus-Hybridomzelle verwendet, die einen gegen den Interleukin 2-Rezeptor gerichteten Antikörper sezerniert.

13. Verfahren nach Anspruch 11 oder 12,
**dadurch gekennzeichnet,**
daß man eine 15 bis 20 bp lange Antikörper Intronsequenz nicht-humanen Ursprungs einführt.

14. Verfahren nach einem der Ansprüche 11 bis 13,
**dadurch gekennzeichnet,**
daß man einen Teil des in der genomischen DNA-Sequenz nicht humanen Ursprungs authentisch sich anschließenden Introns einführt.

15. Verfahren nach einem der Ansprüche 11 bis 14,
**dadurch gekennzeichnet,**
daß man als Splice-Donor-Stelle die Nukleotidfolge GT einführt.

16. Verfahren nach einem der Ansprüche 11 bis 15,
**dadurch gekennzeichnet,**
daß man an das 5'-Ende der letztlich erhaltenen DNA-Sequenz eine Promotorsequenz anfügt, bzw. die Manipulation in einem Vektor durchführt, der in geeigneter Position einen Promotor enthält.

17. Verfahren nach einem der Ansprüche 11 bis 16,
**dadurch gekennzeichnet,**
daß man eine humane genomische DNA-Sequenz verwendet, die für die konstante Region der schweren Kette eines Antikörpers vom Typ IgG kodiert.

18. Verfahren zur Herstellung eines Expressionsvektors,
**dadurch gekennzeichnet,**
daß man in einen zur Expression eines Fremdgens geeigneten Vektor eine rekombinante DNA nach einem der Ansprüche 1 bis 7 bzw. hergestellt nach einem der Ansprüche 11 bis 17 insertiert.

19. Verfahren zur Herstellung eines chimären Antikörpers, dessen variable Regionen nicht-humanen Ursprungs und dessen konstante Regionen humanen Ursprungs sind,
**dadurch gekennzeichnet,**
daß man je einen Expressionsvektor, der eine rekombinante DNA nach einem der Ansprüche 1 bis 7 bzw. hergestellt nach einem der Ansprüche 11 bis 17 enthält, welche für die leichte Kette und einen Expressionsvektor, der eine rekombinante DNA nach einem der Ansprüche 1 bis 7 bzw. hergestellt nach einem der Ansprüche 11 bis 17 enthält, welche für die schwere Kette des Antikörpers kodiert in eine geeignete Wirtszelle einführt, stabile Transformanten isoliert und den Antikörper nach an sich bekannten Methoden aus dem Kulturüberstand der Zellen gewinnt.

20. Verfahren zur Herstellung eines chimären Antikörpers nach Anspruch 19,
**dadurch gekennzeichnet,**
daß man als Wirtszellen Non-producer-Hybridomzellen verwendet.

21. Verfahren nach Anspruch 19,
**dadurch gekennzeichnet,**
daß man die Vektoren pK chim. und pγ chim. in die Wirtszelle einführt.

22. Chimärer Antikörper MAK 179.

17

**23.** Chimärer Antikörper MAK 215.

**24.** Chimärer Antikörper MAK 447.

# Fig.1

ML = multi-linker
V = variable Region
J1 = joining Region 1
C = konstante Region

5'UT = 5' untranslatierte Region

# Fig. 2a

# Fig. 2b

# Fig.3

Eco — BamHI

maus γ1 cDNA
V   DJ   C

ML = multi-linker
V = variable Region
J3 = joining Region 3
D = diversity Region
C = konstante Region
5'UT = 5' untranslatierte Region

ML

pUC γ1

NaeI

Oligonukleotid
Mutagenese

EcoRI — J3 Intron-Sequenz — BamHI

V   D   J₃   C

NotI

pUC γ1-Mut

NaeI

x EcoRI x NotI
Fragment-Isolierung

EcoRI — GT   NotI

5'UT   V   D   J3

Intron

# Fig. 4a

# Fig. 4b

ENH = ENHANCER

= schwere Kette Intron (Maus)